# EUROPÄISCHE PATENTSCHRIFT

(11) **EP 1 448 107 B1**
(45) Veröffentlichungstag und Bekanntmachung des Hinweises auf die Patenterteilung: **20.08.2008**
(21) Anmeldenummer: 02790266.7
(22) Anmeldetag: 27.11.2002
(51) Int. Cl.: A61B 17/22

(54) **STOSS- BZW. DRUCKWELLEN- THERAPIEGERÄT, WIE ZUM BEISPIEL EIN LITHOTRIPTER**
SHOCK WAVE OR COMPRESSION WAVE THERAPY DEVICE, SUCH AS A LITHOTRIPTER
APPAREIL DE THERAPIE PAR ONDES DE CHOC OU PAR ONDES DE COMPRESSION, PAR EXEMPLE, LITHOTRITEUR

(30) Priorität: 29.11.2001 DE 10158519
(43) Veröffentlichungstag der Anmeldung: 25.08.2004
(73) Patentinhaber: Dornier Medtech GmbH, 82234 Wessling (DE)
(72) Erfinder: FORSSMANN, Bernd, 82211 Herrsching (DE)
(74) Vertreter: Grünecker, Kinkeldey, Stockmair & Schwanhäusser Anwaltssozietät
(86) Internationale Anmeldenummer: PCT/DE2002/004351
(87) Internationale Veröffentlichungsnummer: WO 2003/047438

(56) Entgegenhaltungen:
- WO-A-00/53263
- WO-A-01/30281
- WO-A-96/34567
- US-A- 4 819 621
- US-A- 5 358 466

## Beschreibung

Die vorliegende Erfindung betrifft ein Stoss- bzw. Druckwellen-Therapiegerät, beispielsweise zur Durchführung der extrakorporalen Stosswellen-Lithotripsie, deren Therapiekopf unter anderem mit einer Stosswellenquelle ausgerüstet ist, mit der es gelingt, ein im Körper eines Lebewesens befindliches Konkrement berührungslos zu zertrümmern. Darüber hinaus können z.B. auch pathologische Gewebeveränderungen bei Lebewesen - vor allem bei Menschen - nicht invasiv behandelt werden.

Die Stosswellen haben zuweilen beim Durchlaufen des Gewebes von Lebewesen eine hinreichend bekannte nachteilige Folgeerscheinung in Form von Blasenbildungen. Diese Blasen entstehen als unmittelbare Folge der Wechselwirkung der Stosswelle mit der hoppelflüssigkeit oder dem Gewebe. Die Lebensdauer dieser Blasen ist im allgemeinen kurz. Es entstehen aber auch Kavitationskeime und längerlebige Blasen, die die Blasenbildung einer nachfolgenden Stosswelle verstärken. Dieses Phänomen wird von der Fachwelt im weitesten Sinne mit Kavitation bezeichnet.
Die Kavitation wirkt sich in zweifacher Hinsicht nachteilig aus; zum einen bei der Zertrümmerung der Konkremente, die durch die Kavitation beachtlich länger dauert und sich schon aus diesem Grund auch nachteilig auf den Patienten auswirkt.

Inzwischen wurde im Rahmen einschlägiger Forschung erkannt, dass die Kavitation im Stosswellenpfad mit der Intensität der Stosswelle und der Frequenz, mit der die Stosswellen aufeinander folgen, zunimmt. Die Absorption der jeweiligen nachfolgenden Stosswellen im Kavitationsfeld führt auf der einen Seite zu einer Verminderung der Fragmentation der Konkremente, auf der anderen Seite zu einer Zunahme der Nebenwirkungen auf den Patienten, vor allem in Form von schmerzhaften Gewebebeeinträchtigungen.

Auf diese Problematik wird beispielsweise schon im Jahre 1998 hingewiesen, und zwar in einer wissenschaftlichen Abhandlung von Peter Huber und anderen, erschienen in "Phys. Med. Biol. 43 (1998) 3113 - 3128. Printed in UK".

Des weiteren ist aus einem Aufsatz von Ryan Paterson et al, veröffentlicht im "JOURNAL OF UROLOGY, Vol. 165, No. 5, Supplement, June 6, 2001" bekannt, dass die Effizienz der Steinzertrümmerung mit dem Herunterfahren der Pulsfrequenz bei der Stosswellen - Lithotripsie einhergeht.

Schliesslich kamen H. WIKSELL und A.-C. KINN bereits 1995 zu dem Schluss, dass mit der Steigerung der Stosswellenauslösefrequenz im Rahmen der Lithotripsie deren Effizienz abnimmt.

Der Erfindung liegt die Aufgabe zugrunde, die aufgezeigten Nachteile bei Stoss- bzw. Druckwellen-Therapiegeräten der eingangs genannten Art möglichst zu vermeiden und eine Einrichtung vorzuschlagen, die dem Arzt ein möglichst effizientes Arbeiten mit den vorgenannten Therapiegeräten sicherstellt bei möglichst geringen nachteiligen Auswirkungen auf den Patienten.

Gelöst ist diese Aufgabe im wesentlichen durch die kennzeichnenden Merkmale des Patentanspruchs 1. Hiernach ist der die elektrische Energie für die Stoss- bzw. Druckwellenquelle liefernde Stossgenerator eines eingangs genannten Therapiegerätes mit einer Steuereinrichtung verbunden, die die Auslösefrequenz der Stoss- bzw. Druckwellen - im folgenden nur Stosswellen genannt -- als Funktion von deren Pulsenergie steuert, derart, dass höhere Pulsenergien mit geringeren Auslösefrequenzen der Stosswellen korrelieren und umgekehrt.

Die weiteren Patentansprüche betreffen besondere Ausgestaltungen der Verknüpfung der erfindungsgemässen Steuereinrichtung mit dem Stossgenerator.

Durch eine Kopplung der Auslösefrequenz an die Stosswellenenergie kann die pro Zeiteinheit applizierte Energie auf einen kritischen Grenzwert festgelegt werden, der nicht überschritten werden kann bzw. darf; hohe Pulsenergie bestimmt niedrige Auslösefrequenz, und für niedrige Pulsenergien können höhere Auslösefrequenzen gewählt werden. Die Kopplung zwischen Stossgenerator und Steuereinrichtung kann hardware-, software- oder software/hardwaremässig festgelegt werden und zwar obligatorisch oder optional. So kann der Anwender an die Vorgaben gebunden sein oder er wird nur daraufhingewiesen, dass er im gegebenen Falle den kritischen Bereich verlässt. Bei den kritischen Werten handelt es sich in der Praxis um empirisch ermittelte Erfahrungswerte. Wissenschaftlich sinnvoll kann es sein, diesen kritischen Wert aus der sogenannten Halbwertszeit zu bestimmen; unter Halbwertszeit ist die Zeit zu verstehen, nach der die Intensität der Kavitationsblasen auf die Hälfte abgesunken ist. Der kritische Wert kann im Extremfall soweit gesenkt werden, dass eine Wechselwirkung zwischen einander nachfolgenden Stosswellen ausgeschlossen werden kann.

Zur Kontrolle der Intensität der Kavitationsblasen kann über eine Messvorrichtung, beispielsweise über die Messung der Lichtstreuung oder -absorption, an den Kavitationsblasen deren Intensität im Stosswellenfeld der Ankoppelflüssigkeit bzw. des Übertragungsmediums der Stosswelle gemessen werden, um diese Messwerte erfindungsgemäss als Referenz für die Auslösefrequenz zu wählen.
Sinnvoll ist es, auch diese Messwerte mit der Halbwertszeit zu korrelieren, wobei die theoretische Halbwertszeit als Schwellwert dienen kann.

In diesem Zusammenhang kann in an sich bekannter Weise auch hochauflösender Ultraschall verwendet werden, um in vivo im Stosswellenpfad - beispielsweise im Nierenparendym - die Kavitation hinsichtlich ihrer Intensitätsabnahme zu vermessen.

Es ist beispielsweise auch bekannt, dass Stosswellen, die in den menschlichen Körper eingeleitet werden, die Herzaktivität durch Extrasystolie beeinflussen können. In diesem Fall sollten die Stosswellen EKG-getriggert ausgelöst werden. Vorteilhaft ist es, das EKG-Signal aus dem EKG zur Stosswellenauslösung ebenfalls in der Steuereinrichtung als zusätzliche Information zu verarbeiten, dergestalt, dass - wenn die EKG-Auslösefrequenz oberhalb der kritischen Auslösefrequenz liegt - die EKG-Auslösefrequenz reduziert wird, indem beispielsweise jeder zweite, dritte, ..... Triggerpuls ignoriert wird, so dass im Mittel die gewünschte Auslösefrequenz erreicht wird.
Eine derartige Vorsichtsmassnahme kann unter Umständen Menschenleben retten.

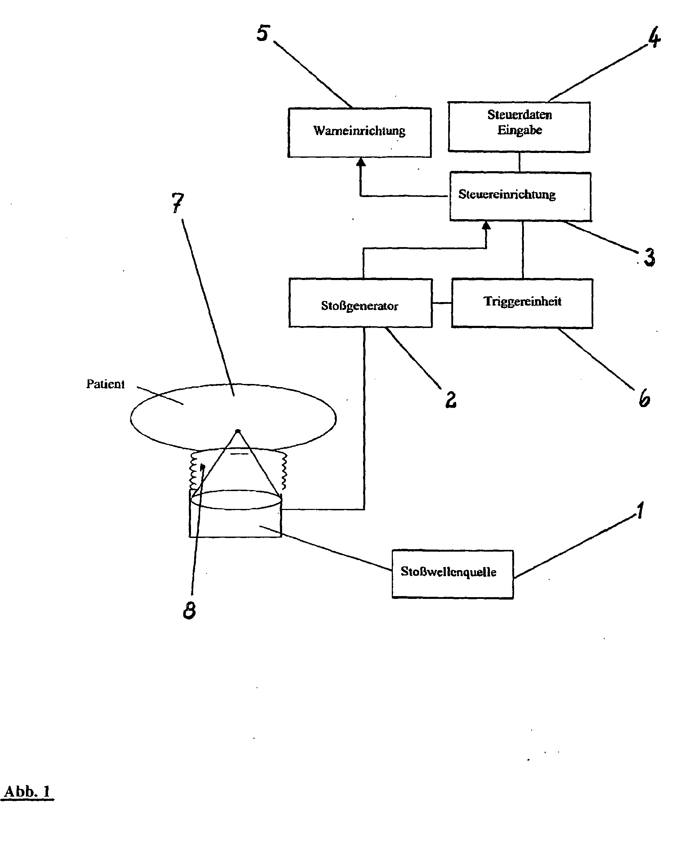

In den Abbildungen ist die Erfindung zeichnerisch erläutert. Die Abbildungen 1 bis 3 zeigen drei unterschiedliche Block-Diagramme zur Veranschaulichung der Verknüpfung der erfindungsgemässen Steuereinrichtung mit einem Stossgenerator einer Stosswellenquelle.

Gemäss der Abbildung 1 ist eine Stosswellenquelle 1 über einen Stossgenerator 2 mit einer Steuereinrichtung 3 verbunden, in welche einzuhaltende Steuerdaten als Grenzwerte vor allem hinsichtlich der Pulsenergie und der Auslösefrequenzen der Stosswellen über eine Eingabeeinrichtung 4 eingegeben werden können. Die erfindungsgemässe Steuereinrichtung 3 kann somit dem behandelnden Arzt über eine an die Steuereinrichtung 3 angeschlossene Warneinrichtung 5 durch Warnsignale wie Blinklicht, Tonsignale und dergleichen anzeigen, dass bestimmte Grenzwerte bald erreicht oder bereits überschritten werden. Grundsätzlich kann die Verbindung der Steuereinrichtung 3 mit dem Stossgenerator 2 auch so eingerichtet werden, dass die Grenzwerte für die Pulsenergie und die Auslösefrequenzen der Stosswellen automatisch eingehalten werden und damit von dem behandelden Arzt zwangsläufig eingehalten werden müssen. Aus schaltungstechnischen Gründen ist der Stossgenerator 2 mit der Steuereinrichtung 3 zusätzlich über eine Triggereinheit 6 verbunden. Zwischen dem Patienten 7 und der Stosswellenquelle 1 befindet sich ein Koppelbalg 8.

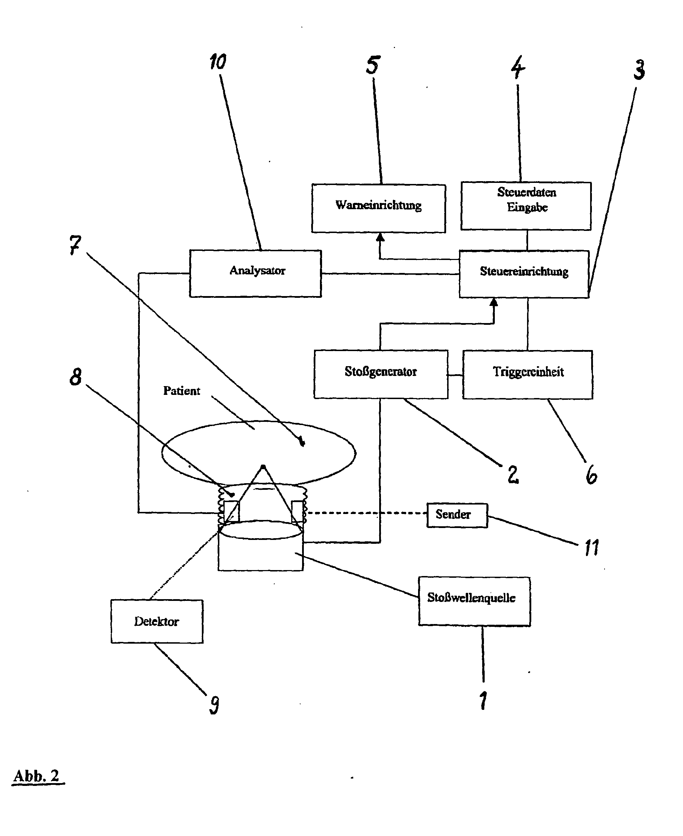

Die Abbildung 2 zeigt ein weiteres Block-Diagramm für eine mögliche andere Einbindung der erfindungsgemässen Steuereinrichtung 3 in das Umfeld eines Stosswellen-Therapiegerätes.

Hier werden die Kavitationsblasen über einen Detektor 9 im Koppelkissen 8 erfasst und die betreffenden Signale in einem Analysator 10 ausgewertet. Diese Art, Steuerdaten zu ermitteln stützt sich auf die Tatsache, dass die Stärke der Kavitationsblasenbildung innerhalb des Koppelkissens 8 zwischen der Stosswellenquelle 1 und dem Patienten 7 mit der Kavitationsblasenbildung im Körper des Patienten 7 korreliert.

Über den Eingabekanal 4 der Steuereinrichtung 3 können zusätzliche Informationen zur Verfügung gestellt werden, wie zum Beispiel ein Algorithmus, wie die analysierte Kavitation zu bewerten ist und die Auslösefrequenz zu steuern ist.

Zum Erfassen der Kavitationsblasen kann beispielsweise die Lichtstreuung oder -absorption mit Hilfe eines Senders 11 in Form einer Lichtquelle und eines Detektors 9 verwendet werden. Die Anwendung von Ultraschall wäre dafür ebenfalls geeignet.

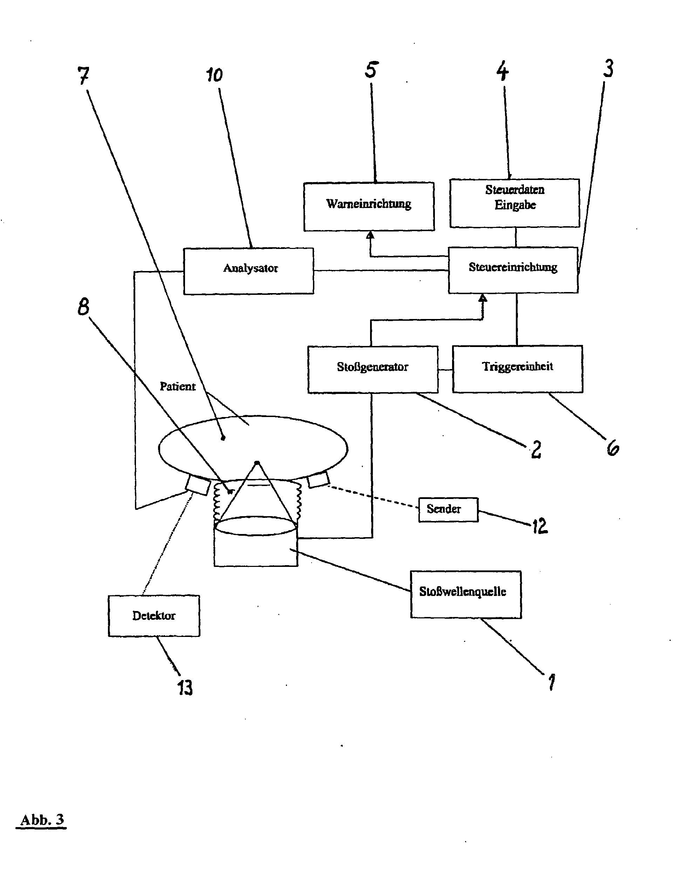

Gemäss dem Block-Diagramm in Abbildung 3 messen ein Sender 12 und Detektor 13 die Kavitation direkt im Körper eines Patienten 7. Für diese Messung kommt vorzugsweise Ultraschall in Betracht. Sender 12 und Detektor( Empfänger) 13 können vorteilhafterweise in ein und demselben Gehäuse untergebracht sein.

Über die Steuereinrichtung 3 kann auch hier mittels einer Warneinrichtung 5 dem Arzt signalisiert werden, sobald er sich in kritischen Bereichen befindet. Die Warneinrichtung 5 kann auch dazu benutzt werden, die Auslösefrequenz in Korrelation zur Pulsenergie der Stosswelle zwangsweise festzusetzen.

## Patentansprüche

1. Stoss- bzw. Druckwellen-Therapiegerät, wie zum Beispiel ein Lithotripter, dessen Therapiekopf u.a. mit einer Stosswellenquelle ausgerüstet ist, **dadurch gekennzeichnet, dass** die Stosswellenquelle (1) über einen Stossgenerator (2) mit einer Steuereinrichtung (3) verbunden ist, die die Auslösefrequenz der Stosswellen als Funktion von deren Pulsenergie steuert, derart, dass höhere Pulsenergien mit geringeren Auslösefrequenzen der Stosswellen korrelieren und umgekehrt.

2. Stoss- bzw. Druckwellen-Therapiegerät nach Anspruch 1, **dadurch gekennzeichnet, dass** in der Steuereinrichtung (3) die Korrelation zwischen Auslösefrequenz und Pulsenergie der Stosswellen soft- und/ oder hardwaremässig festlegbar ist.

3. Stoss- bzw. Druckwellen-Therapiegerät nach den Ansprüchen 1 und 2, **dadurch gekennzeichnet, dass** die Auslösefrequenz der Stosswellen unter einem kritischen Wert, zum Beispiel der sogenannten Halbwertszeit liegt.

4. Stoss- bzw. Druckwellen-Therapiegerät nach den Ansprüchen 1 bis 3,
**dadurch gekennzeichnet, dass** die Steuereinrichtung (3) mit einer Warneinrichtung (5) verbunden ist.

5. Stoss- bzw. Druckwellen-Therapiegerät nach einem oder mehreren der Ansprüche 1 bis 4, **dadurch gekennzeichnet, dass** die Steuereinrichtung (3) automatisch nach einem festen Programm gemäss den eingegebenen Grenzwerten für Pulsenergie und Auslösefrequenz der Stosswellen den Stossgenerator (2) steuert.

6. Stoss- bzw. Druckwellen-Therapiegerät nach einem oder mehreren der Ansprüche 1 bis 5 , **dadurch gekennzeichnet, dass** die Stosswellenquelle (1) ausschliesslich über die Steuereinrichtung (3) einschaltbar und nur über diese funktionsfähig ist, solange die Steuereinrichtung (3) entsprechend den vorgegebenen Korrespondenzkurven aus Auslösefrequenz und Pulsenergie die Therapie steuert.

7. Stoss- bzw. Druckwellen-Therapiegerät nach einem oder mehreren der Ansprüche 1 bis 6, **dadurch gekennzeichnet, dass** in dem mit der Stosswellenquelle (1) verbundenen Koppelkissen (8) ein Detektor (9) angeordnet ist, der mit einem Sender (11) korrespondiert zur Überprüfung der Intensität der Kavitationsblasen in der Ankoppelflüssigkeit bzw. im Übertragungsmedium, und der die detektierten Signale über einen Analysator (10) an die Steuereinrichtung (3) weiterleitet.

8. Stoss- bzw. Druckwellen-Therapiegerät nach Anspruch 7, **dadurch gekennzeichnet, dass** der Sender (11) als Lichtquelle ausgebildet ist, sodass von dem Detektor (9) die Lichtstreuung und/oder -absorption der Kavitationsblasen detektiert und über den Analysator (10) an die Steuereinrichtung (3) weitergeleitet werden können.

9. Stoss- bzw. Druckwellen-Therapiegerät nach Anspruch 8, **dadurch gekennzeichnet, dass** der Sender (11) als Ultraschallgeber ausgebildet ist, und dass die detektierten Signale analog zu Anspruch 8 an die Steuereinrichtung (3) weitergeleitet werden.

10. Stoss- bzw. Druckwellen-Therapiegerät nach einem oder mehreren der Ansprüche 1 bis 7, **dadurch gekennzeichnet, dass** die Intensität der Kavitationsblasen mittels eines Senders (12) und eines Detektors (13) direkt im Körper eines Patienten (7) detektiert und über den Analysator (10) an die Steuereinrichtung (3) weitergeleitet wird.

11. Stoss- bzw. Druckwellen-Therapiegerät nach Anspruch 10, **dadurch gekennzeichnet, dass** der Sender (12) als Ultraschallgeber ausgebildet ist.

## Claims

1. Shockwave or pressure-wave type therapeutic apparatus, such as a lithotripter, whose therapy head is inter alia equipped with a shockwave source, **characterized in that** the shockwave source (1) is connected via a shock generator (2) to a control means (3) which controls the release frequency of the shockwaves as a function of the pulse energy thereof such that increased pulse energies correlate with lower release frequencies of the shockwaves, and vice versa.

2. Shockwave or pressure-wave type therapeutic apparatus according to claim 1, **characterized in that** in the control means (3) the correlation between release frequency and pulse energy of the shockwaves can be set according to software and/or hardware.

3. Shockwave or pressure-wave type therapeutic apparatus according to claims 1 and 2, **characterized in that** the release frequency of the shockwaves is below a critical value, e.g. the so-called half-life period.

4. Shockwave or pressure-wave type therapeutic apparatus according to claims 1 to 3, **characterized in that** the control means (3) is connected to an alarm means (5).

5. Shockwave or pressure-wave type therapeutic apparatus according to one or several of claims 1 to 4, **characterized in that** the control means (3) automatically controls the shock generator (2) according to a fixed program according to the limit values entered for pulse energy and release frequency of the shockwaves.

6. Shockwave or pressure-wave type therapeutic apparatus according to one or several of claims 1 to 5, **characterized in that** the shockwave source (1) can exclusively be switched on via the control means (3) and is only operative via said means as long as said control means (3) controls the therapy according to the predetermined correspondence curves of release frequency and pulse energy.

7. Shockwave or pressure-wave type therapeutic apparatus according to one or several of claims 1 to 6, **characterized in that** a detector (9) is arranged in the coupling pad (8) connected to said shockwave source (1), said detector corresponding with a transmitter (11) for checking the intensity of the cavitation bubbles in the coupling liquid or in the transfer medium, and said detector passing on the detected signals via an analyzer (10) to the control means (3).

8. Shockwave or pressure-wave type therapeutic apparatus according to claim 7, **characterized in that** the transmitter (11) is designed as a light source so that light scattering and/or absorption of the cavitation bubbles is detected by the detector (9) and can be passed on via the analyzer (10) to the control means (3).

9. Shockwave or pressure-wave type therapeutic apparatus according to claim 8, **characterized in that** the transmitter (11) is designed as an ultrasonic generator, and that the detected signals are passed on - by analogy to claim 8 - to the control means (3).

10. Shockwave or pressure-wave type therapeutic apparatus according to one or several of claims 1 to 7, **characterized in that** the intensity of the cavitation bubbles is detected by means of a transmitter (12) and a detector (13) directly in the body of a patient (7) and is passed on via the analyzer (10) to the control means (3).

11. Shockwave or pressure-wave therapeutic apparatus according to claim 10, **characterized in that** the transmitter (12) is designed as an ultrasonic generator.

## Revendications

1. Appareil de thérapie par ondes de choc ou ondes de compression, tel qu'un lithotriteur, dont la tête est équipée, entre autres, d'une source d'ondes de chocs, **caractérisé en ce que** la source d'ondes de choc (1) est reliée par l'intermédiaire d'un générateur de chocs (2) à un dispositif de commande (3), qui commande la fréquence de déclenchement des ondes de choc en fonction de l'énergie impulsionnelle, de telle sorte que des énergies impulsionnelles plus élevées sont en corrélation avec des fréquences de déclenchement plus faibles et inversement.

2. Appareil de thérapie par ondes de choc ou ondes de compression selon la revendication 1, **caractérisé en ce que** la corrélation entre la fréquence de déclenchement et l'énergie impulsionnelle des ondes de choc peut être définie dans le dispositif de commande (3) par un logiciel et/ou par un matériel.

3. Appareil de thérapie par ondes de choc ou ondes de compression selon les revendications 1 et 2, **caractérisé en ce que** la fréquence de déclenchement des ondes de choc se situe en dessous d'une valeur critique, telle que la durée à mi-crête.

4. Appareil de thérapie par ondes de choc ou ondes de compression selon une ou plusieurs des revendications 1 à 3, **caractérisé en ce que** le dispositif de commande (3) est relié à un dispositif d'alarme (5).

5. Appareil de thérapie par ondes de choc ou ondes de compression selon une ou plusieurs des revendications 1 à 4, **caractérisé en ce que** le dispositif de commande (3) commande automatiquement le générateur de chocs (2) selon un programme fixe conformément à des valeurs limites entrées pour l'énergie impulsionnelle et la fréquence de déclenchement des ondes de choc.

6. Appareil de thérapie par ondes de choc ou ondes de compression selon une ou plusieurs des revendications 1 à 5, **caractérisé en ce que** la source d'ondes de choc (1) peut être activée uniquement par l'intermédiaire du dispositif de commande (3) et est apte à fonctionner uniquement par l'intermédiaire de ce dernier, tant que le dispositif de commande (3) commande la thérapie conformément aux courbes de correspondance définies à partir de la fréquence de déclenchement et de l'énergie impulsionnelle.

7. Appareil de thérapie par ondes de choc ou ondes de compression selon une ou plusieurs des revendications 1 à 6, **caractérisé en ce que** dans le coussin de couplage (8), relié à la source d'ondes de choc (1), est monté un détecteur (9), lequel coopère avec un émetteur (11) pour contrôler l'intensité des bulles de cavitation dans le liquide de couplage ou dans le fluide de transmission, et par lequel les signaux détectés sont transmis par l'intermédiaire d'un analyseur (10) vers le dispositif de commande (3).

8. Appareil de thérapie par ondes de choc ou ondes de compression selon la revendication 7, **caractérisé en ce que** l'émetteur (11) est réalisé sous la forme d'une source lumineuse, de telle sorte que la dispersion et/ou l'absorption de lumière par les bulles de cavitation peuvent être détectées par le détecteur (9) et peuvent être transmises par l'intermédiaire de l'analyseur (10) vers le dispositif de commande (3).

9. Appareil de thérapie par ondes de choc ou ondes de compression selon la revendication 8, **caractérisé en ce que** l'émetteur (11) est réalisé sous la forme d'un transmetteur à ultrasons, et **en ce que** les signaux détectés sont transmis, de manière analogue à la revendication 8, vers le dispositif de commande (3).

10. Appareil de thérapie par ondes de choc ou ondes de compression selon une ou plusieurs des revendications 1 à 7, **caractérisé en ce que** l'intensité des bulles de cavitation est détectée directement dans le corps d'un patient (7) au moyen d'un émetteur (12) et d'un détecteur (13) et est transmise par l'intermédiaire de l'analyseur (10) vers le dispositif de commande (3).

11. Appareil de thérapie par ondes de choc ou ondes de compression selon la revendication 10, **caractérisé en ce que** l'émetteur (12) est réalisé sous la forme d'un transmetteur à ultrasons.
